# EUROPEAN PATENT APPLICATION

(11) **EP 3 260 150 A1**
(43) Date of publication of application: **27.12.2017**
(21) Application number: 16175312.4
(22) Date of filing: 20.06.2016
(51) Int. Cl.: A61M 5/32

(54) **IMPROVED NEEDLE DESTROYER**

(71) Applicant: Besafe S.r.l., 479922 Rimini (RN) (IT)
(72) Inventor: CIOPPI, Simone, 61022 MONTECCHIO DI VALLE FOGLIA (PU) (IT)
(74) Representative: Baldi, Claudio

(57) **Abstract**

A needle destroyer comprising a body (1) provided with a hole (11) used to insert a needle to be cut, a cutting blade (2) slidingly supported by the body (1) in order to cut the needle inserted in the hole (11) of the body, and an electric actuator (40) in order to actuate said cutting blade.

## Description

The present patent application for industrial invention relates to an improved needle destroyer.

US4531437 discloses a rotary needle and syringe destructor comprising a body provided with an opening used to insert a needle to be cut and a cutting blade intended to cut the needle inserted in the opening of the body. A handle is supported by the body and is connected to the cutting blade by means a lever mechanism. The handle is intended to be manually actuated by a user to actuate the cutting blade. Therefore the actuation of the cutting blade requires the manual action of the user, with an inevitable physical effort as well as an increase in the time needed to cut a needle. In fact, the user must insert the needle to be cut manually inside the hole of the body and move the handle from an initial position to a final position in order to actuate the cutting blade. Moreover, the user must exert a force on the handle in order to cut the needle. To reduce the effort of the user, the handle could be connected to the cutting blade with a force demultiplier device; however, such a force demultiplier force device would be complicated, cumbersome and not very reliable.

US5692687 discloses an apparatus used to disinfect medical waste, such as syringes or the like. Such an apparatus comprises a disinfecting unit comprising a housing with an upper opening in order to allow a user to manually insert medical devices to be disinfected inside the disinfecting unit. Such an apparatus is not provided with a cutting blade to cut needles.

The purpose of the present invention is to overcome the drawbacks of the prior art by providing a needle destroyer that is efficient, reliable, simple and fast to use, and eliminates the need of making a manual effort in order to cut the needle.

Another purpose of the present invention is to disclose such a needle destroyer able to eliminate the need for a manual action by the user.

These purposes are achieved according to the independent claim 1.

Advantageous embodiments appear from the dependent claims.

The needle destroyer of the invention comprises:
- a body provided with a hole used to insert a needle to be cut;
- a cutting blade supported by the body and intended to cut the needle inserted in such a hole;
- actuation means to actuate said cutting blade;
- an internally hollow receptacle removably connected to the body;
said receptacle comprising an opening intended to be disposed in register with the hole of the body, in such a way that the needle inserted in the hole of the body can fall inside the receptacle through the opening of the receptacle.

The actuation means of the cutting blade comprise an electric actuator.

The advantages of the needle destroyer of the invention are evident, meaning that the provision of an electric actuator intended to actuate the cutting blade makes it possible to automate the operation of the needle destroyer, thus eliminating the need for actuating the cutting blade manually and reducing the effort and the time needed to cut a needle.

For the sake of clarity, the description of the needle destroyer according to the invention continues with reference to the attached drawings, which have a merely illustrative, not limiting value, wherein:
Fig. 1 is an exploded axonometric view of a body and a receptacle of the needle destroyer according to the invention, after removing one wall of the body;
Fig. 2 is an axonometric view that shows the upper wall of the body and an electric actuator connected to a cutting blade in idle position;
Fig. 3 is the same view as Fig. 2, except for the fact that it shows the cutting blade in operating position;
Fig. 4 is an axonometric view of an upper wall of the receptacle of the needle destroyer according to the invention;
Fig. 5 is a perspective view of two mobile partitions of the needle destroyer according to the invention;
Fig. 6 is a top view of the lid of Fig. 4 with the mobile partitions of Fig. 5, wherein the mobile partitions are in closed position;
Fig. 7 is the same view as Fig. 6, except for the fact that the mobile partitions are in opening position; and
Fig. 8 is a perspective view of the receptacle coupled with the body of the needle destroyer according to the invention.

With reference to the figures, the needle destroyer according to the invention is disclosed, which is generally indicated with reference numeral 100.

Although the following description refers to a needle, such as a disposable syringe, the needle destroyer the invention can be also used to cut the tip of surgical instruments, such as surgical knives and the like.

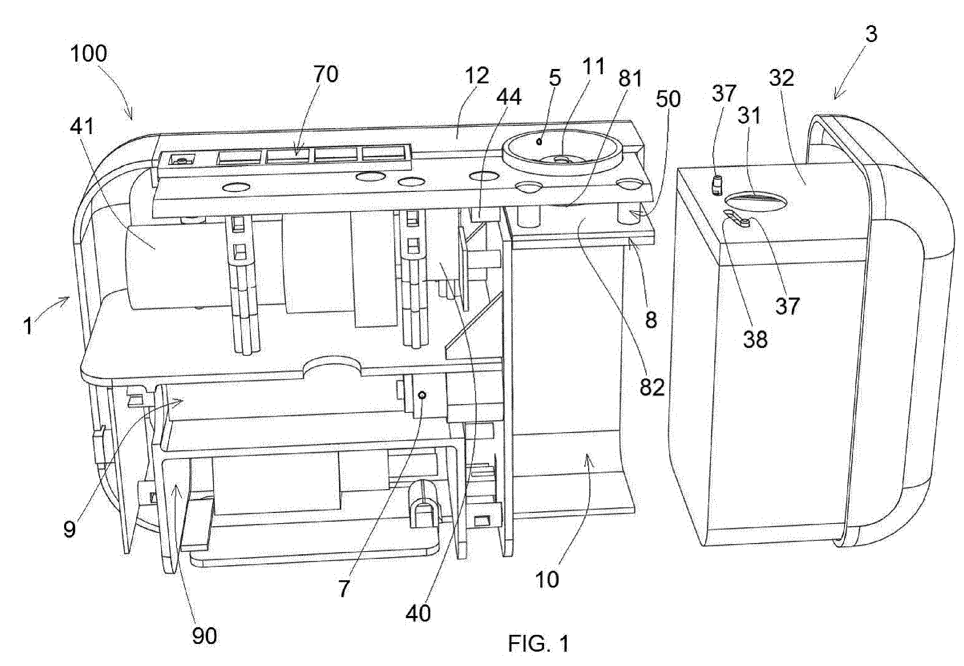

With reference to Fig. 1, the needle destroyer (100) comprises a body (1) and a receptacle (3) intended to be removably connected to the body (1).

The body (1) comprises an upper wall (12) wherein a through hole (11) is obtained and intended to insert a needle to be cut.

The body (1) comprises a housing (10) obtained under the hole (11) to receive the receptacle (3). The receptacle (3) comprises an upper wall (32) wherein an opening (31) is obtained and intended to be disposed in register with the hole (11) of the body, when the receptacle (3) is contained in the housing (10) of the body. In such a way, after being cut, a needle can fall in the receptacle (3).

The receptacle (3) can be coupled with the body (1) in snap-in coupling mode.

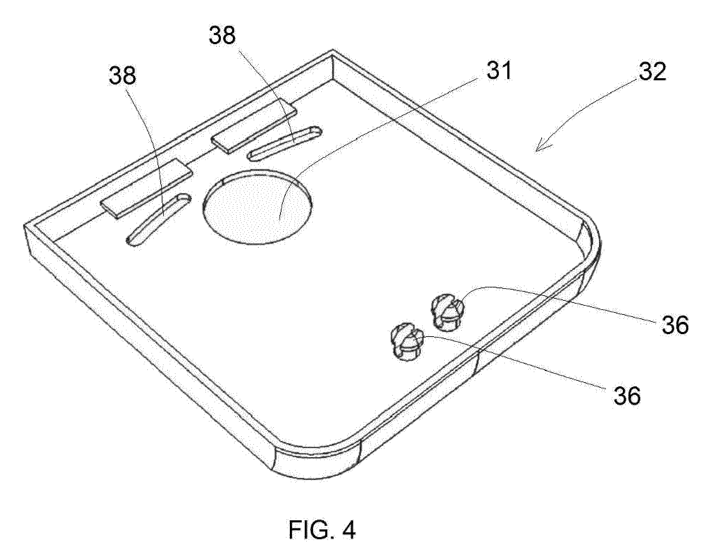

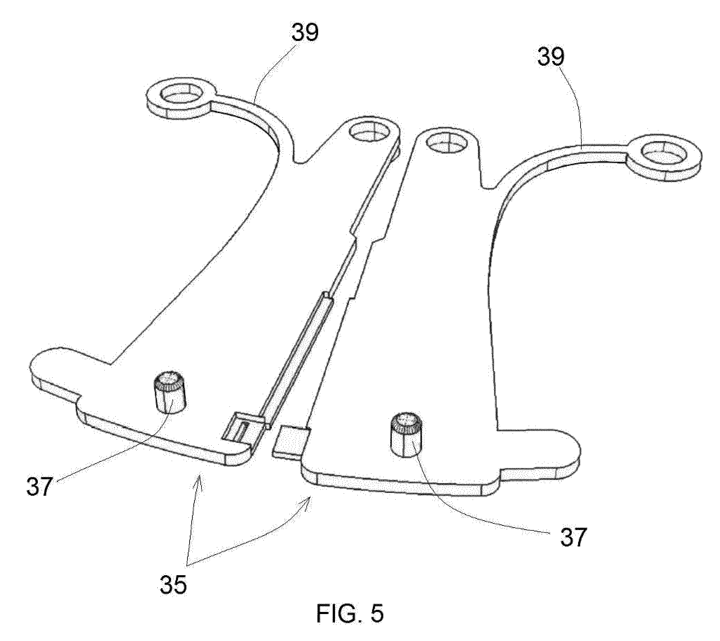

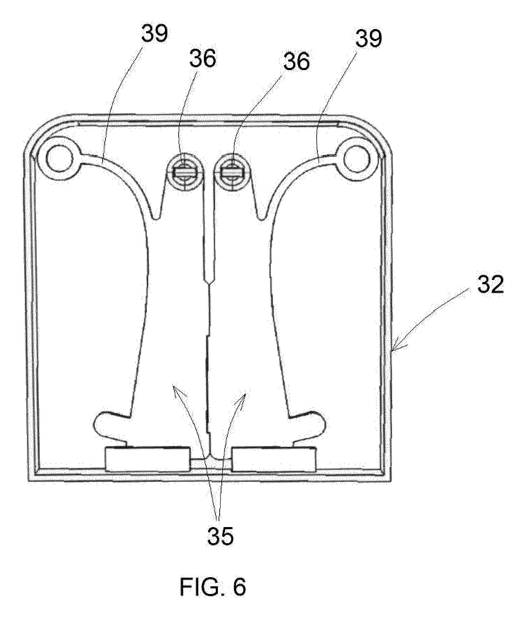

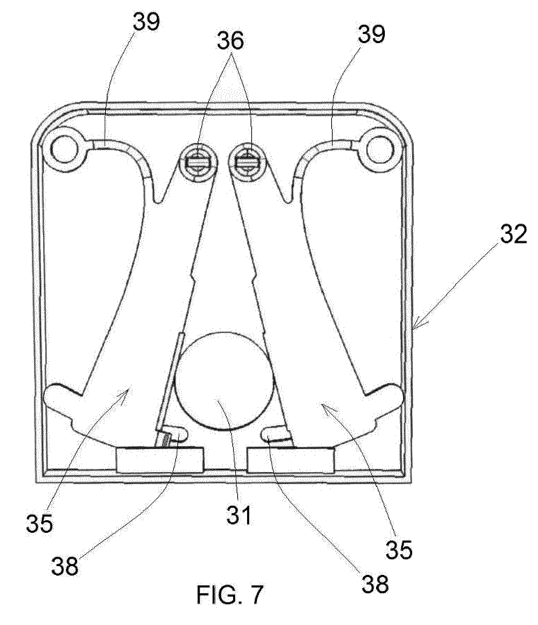

With reference to Figs. 4 - 7, the receptacle (3) comprises two mobile partitions (35) disposed under the upper wall (32) of the receptacle. Each mobile partition (35) is hinged to the upper wall (32) of the receptacle in a hinge (36) with vertical axis. In such a way, by rotating around the hinges (36), the mobile partitions (35) can go from a closing position (Fig. 6) wherein the edges of the mobile partitions (35) are in contact in order to close the opening (31) of the receptacle, to an opening position (Fig. 7) wherein the edges of the mobile partitions (35) are mutually spaced in order to open the opening (31) of the receptacle.

Elastic wings (39) are connected to the mobile partitions (35), said elastic wings (39) acting as spring means to hold the mobile partitions (35) in closing position (Fig. 6) Therefore, when the receptacle (3) is not coupled inside the housing (10) of the body, the opening (31) of the receptacle is closed by the mobile partitions (35).

With reference to Fig. 1, a plate (8) is disposed under the upper wall (12) of the body in correspondence of the hole (11). The plate (8) is spaced from the upper wall (12) by means of spacing columns (80). In view of the above, an air gap (82) is generated between the plate (8) and the upper wall (12) and intended to make a cutting blade (2) pass through.

The plate (8) has a hole (81) disposed in register with the hole (11) of the body to let the needle pass through after being cut.

Under the plate (8) the housing (10) is obtained and intended to receive the receptacle (3). Therefore, the hole (81) of the plate is disposed in register with the opening (31) of the receptacle in order to let the needle fall in the receptacle after being cut.

Actuation means (D) are provided on the mobile partitions (35), on the receptacle (3) and on the plate (8) fixed to the body in order to actuate the mobile partitions (35) from the closing position to the opening position, when the receptacle (3) is coupled inside the housing (10) of the body.

The actuation means (D) comprise a pin (37) that protrudes in upper position from each mobile partition (35) in order to be slidingly engaged inside a guide slot (38) obtained on the upper wall (32) of the receptacle in proximity to the opening (31) of the receptacle. In such a way, each pin (37) of the mobile partitions protrudes in upper position from the upper wall (32) of the receptacle through the guide slot (38), as shown in Fig. 1.

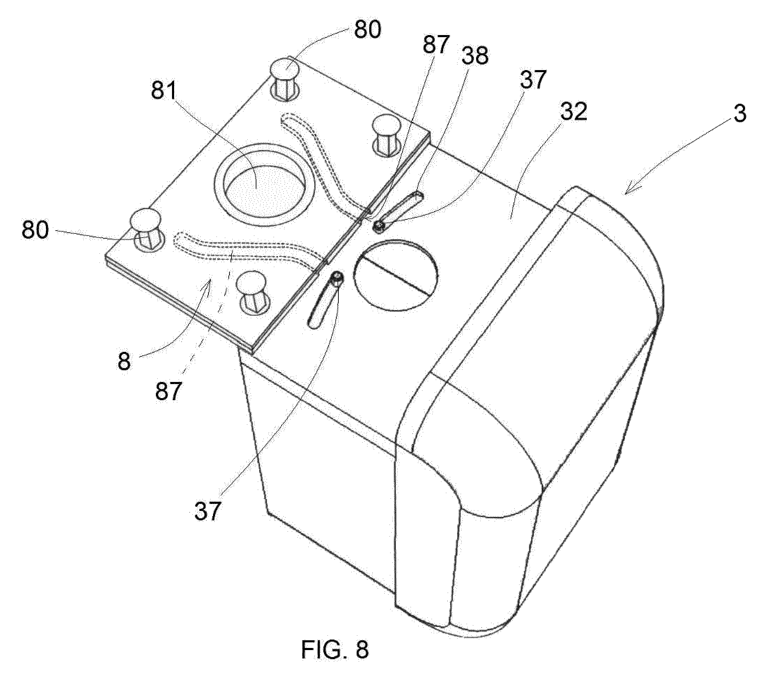

With reference to Fig. 8, when the receptacle (3) is coupled inside the housing (10) of the body, each pin (37) of the mobile partitions is slidingly engaged also inside a guide notch (87) obtained on the plate (8) of the body of the needle destroyer in proximity to the hole (81) of the plate.

The guide notches (87) of the plate of the needle destroyer act as cams for the pins (37) of the mobile partitions, in such a way to let the mobile partitions (35) open under the action of said elastic wings (39) of the mobile partitions.

Although two mobile partitions (35) are shown in the figures, the needle destroyer (100) can comprise only one mobile partition intended to close and open the opening (31) of the receptacle.

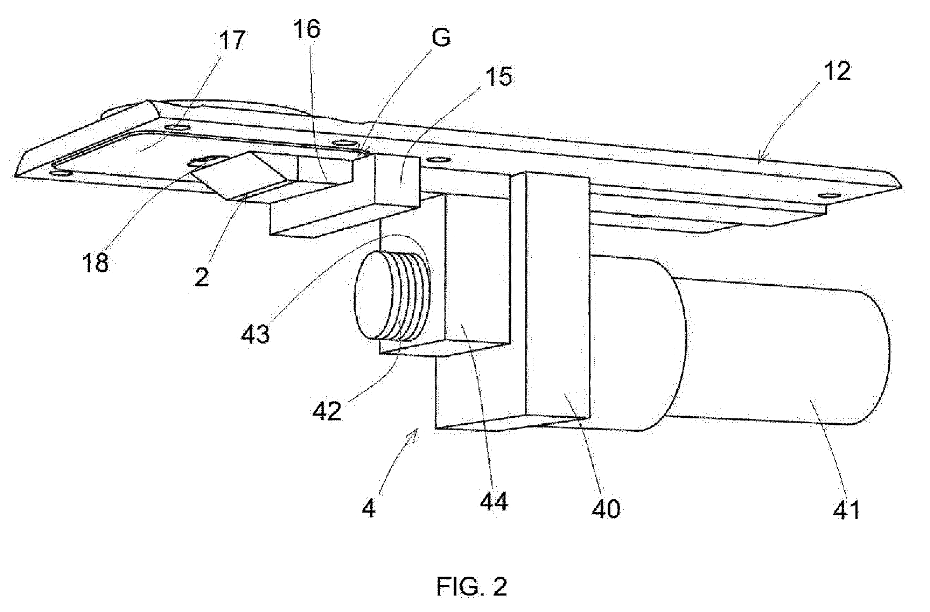

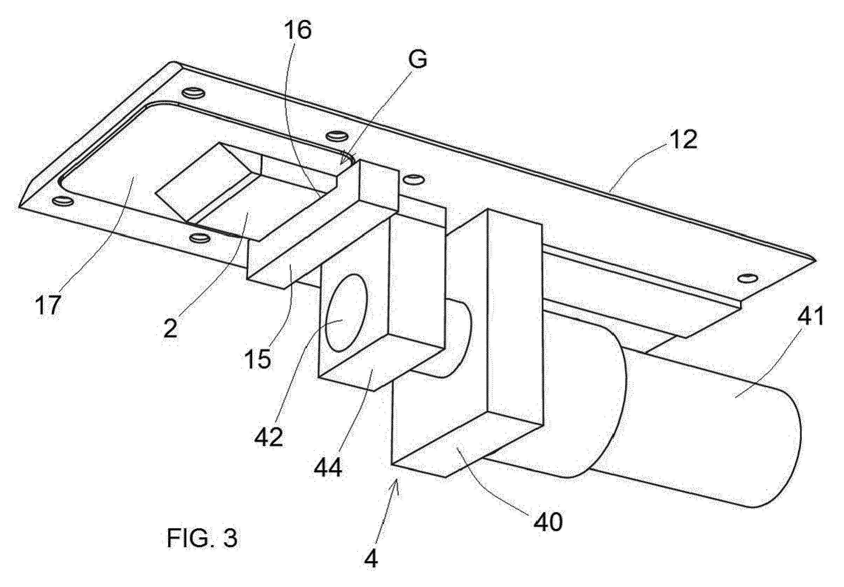

With reference to Figs. 2 and 3, a cutting blade (2) is disposed under the upper wall (12) of the body and is slidingly mounted with respect to the upper wall (12) of the body.

A plate-shaped counter-blade (17) is mounted under the upper wall (12) of the body. The counter-blade (17) has a hole (18) in register with the hole (11) of the body. In such a way the cutting blade (2) slides on the counter-blade (17). Therefore the needle is cut between the edge of the cutting blade (2) and the edge of the hole (18) of the counter-blade (17), like with scissors.

Actuation means (4) actuate the cutting blade (2) in order to cut a needle inserted in the hole (11) of the body.

In Fig. 2 the cutting blade (2) is in idle position, with the tip of the blade proximal to the hole (11) of the body and ready to cut a needle inserted in the hole (11) of the body. In Fig. 3 the cutting blade (2) is in operating position, with the tip of the blade under the hole (11) of the body, that is to say in a position in which it has cut the needle inserted in the hole (11) of the body.

The actuation means (4) comprise an electric actuator (40) fixed to the upper wall (12) of the body and connected to the cutting blade (2) in order to actuate the cutting blade.

The electric actuator (40) comprises an electrical motor (41) that actuates an endless screw (42) with axis of rotation in parallel position to the upper wall (12) of the body and to the cutting blade (2).

A support (44) is connected to the cutting blade (2) and protrudes in lower position from the cutting blade (2). A female screw (43) is obtained in the support (44), said endless screw (42) being engaged in the female screw (43). In such a way, a rotation of the endless screw (42) causes a translation of the support (44) joined with the cutting blade (2) and, consequently, a translation of the cutting blade (2).

Guide means (G) guide the translation of the cutting blade (2). The guide means (G) comprise a bracket (15) fixed to the upper wall (12) of the body and protruding in lower position from the upper wall (12) of the body. Said bracket (15) is disposed between the support (44) and the hole (11) of the body of the needle destroyer. The bracket (15) comprises an opening (16) wherein the cutting blade (2) slides. The opening (16) has the same width as the cutting blade (2).

With reference to Fig. 1, the needle destroyer (100) comprises a presence detector (5) disposed on the upper wall (12) of the body, in proximity to the hole (11) in order to detect the presence of a needle inserted in the hole (11). The presence sensor (5) is connected to the electric actuator (40) in order to actuate the electric actuator when the presence sensor (5) detects the presence of the needle in the hole (11) of the body.

Advantageously, the presence sensor (5) comprises a button intended to be compressed by a syringe, when the needle of the syringe is completely inserted in the hole (11) of the body.

The needle destroyer (100) comprises a full sensor (7) disposed on the body (1) or in the receptacle (3) to detect when the receptacle (3) is full of needles and needs to be emptied.

A warning device (70) is connected to the full sensor (7) to generate a warning signal when the full sensor (7) detects that the receptacle (3) is full of needles that have been cut. The warning device (70) can be of acoustic and/or visual or luminous type and can comprise, for example, LEDs.

A battery (9) is disposed in the body (1) and is connected to the electric actuator (40) in order to power the electrical actuator.

A container (90) is disposed in the body, under the electric actuator (40) to contain a power supply cable intended to be connected to the electrical mains to charge the battery (9) or to power the electric actuator (40) directly from the electrical mains.

## Claims

1. Needle destroyer comprising:
- a body (1) provided with a hole (11) to insert a needle to be cut;
- a cutting blade (2) slidingly supported by said body (1) and adapted to cut the needle inserted in such a hole (11) of the body;
- actuation means (4) to actuate said cutting blade (2);
- an internally hollow receptacle (3) removably connected to the body (1); said receptacle (3) comprising an opening (31) intended to be disposed in register with said hole (11) of the body (1), in such a way that the needle inserted in the hole (11) of the body can fall inside the receptacle (3) through the opening (31) of the receptacle.
**characterized in that**
said actuation means (4) of said cutting blade (2) comprise an electric actuator (40).

2. The needle destroyer of claim 1, wherein said electric actuator (40) comprises:
- an electric motor (41) supported by the body (1);
- a female screw (43) joined to the cutting blade (2);
- an endless screw (42) connected to said electrical motor (41) in order to be driven into rotation by said electrical motor (41); said endless screw (42) being intended to engage with said female screw (43) in such a way that a rotation of the endless screw (42) determines a translation of the cutting blade (2).

3. The needle destroyer of claim 2, wherein said electrical actuator (40) also comprises a support (44) wherein said female screw (43) is obtained; said support (44) being joined to the cutting blade (2).

4. The needle destroyer of any one of the preceding claims, comprising guide means (G) intended to guide the translation of the cutting blade (2) with respect to the blade (1).

5. The needle destroyer of claim 4, wherein said guide means (G) comprise a bracket (15) protruding in lower position from the upper wall (12) of the body and comprising an opening (16) wherein the cutting blade (2) is slidingly engaged.

6. The needle destroyer according to any one of the preceding claims, also comprising a presence sensor (5) supported by the body (1) and connected to the electric actuator (40); said presence sensor (5) being disposed in such a way to detect the presence of a needle inserted in said hole (11) of the body; said presence sensor (5) being configured in such a way to enable the electric actuator (40) to actuate said cutting blade (2) when the presence sensor (5) detects the presence of a needle in the hole (11) of the body (1).

7. The needle destroyer of claim 6, wherein said presence sensor (5) is disposed in correspondence of the hole (11) of the body and comprises a button intended to be compressed by a syringe when the needle of the syringe is completely inserted in the hole (11) of the body.

8. The needle destroyer of any one of the preceding claims, also comprising:
- a full sensor (7) mounted on the body (1) or in the receptacle (3) to detect when the receptacle (3) is full of needles and needs to be emptied,
- a starter (70) connected to the full sensor (7) to generate a warning signal when the full sensor (7) detects that the receptacle (3) is full of needles.

9. The needle destroyer of any one of the preceding claims, also comprising:
- a plate (8) disposed under the upper wall (12) of the body in correspondence of the hole (11); said plate (8) comprising a hole (81) disposed in register with the hole (11) of the body;
- at least one mobile partition (35) disposed on the receptacle (3) in proximity to said opening (31) of the receptacle (3), said mobile partition (35) being in a closing position wherein it closes the opening (31) of the receptacle (3) when the receptacle (3) is not coupled with the body (1);
- actuation means (D) provided in the receptacle (3), in the mobile partition (35) and in said plate (8) to actuate said at least one mobile partition (35) from the closing position to an opening position wherein said mobile partition (35) opens said opening (31) of the receptacle when the receptacle (3) is coupled with the body (1).

10. The needle destroyer of claim 9, wherein said actuation means (D) of said at least one mobile partition comprise a pin (37) that protrudes in upper position from said mobile partition (35) and is slidingly engaged inside a guide slot (38) obtained on said receptacle (3) and inside a guide notch (87) obtained in said plate (8).

11. The needle destroyer of claim 9 or 10, comprising two mobile partitions (35) hinged to the receptacle (3) at corresponding hinges (36) with a vertical axis in such a way to go from a closing position wherein the edges of the mobile partitions (35) are in contact and close the opening (31) of the receptacle (3), to an opening position wherein the edges of the mobile partitions (35) are mutually spaced and open the opening (31) of the receptacle.

12. The needle destroyer of claim 9, comprising spacing columns (80) disposed between said upper wall (12) of the body and said plate (8), in such a way to generate an air gap between the plate (8) and the upper wall (12) of the body in order to let the cutting blade (2) pass through.
